# EUROPEAN PATENT SPECIFICATION

(11) **EP 3 068 750 B1**
(45) Date of publication and mention of the grant of the patent: **22.04.2020**
(21) Application number: 14798888.5
(22) Date of filing: 14.11.2014
(51) Int. Cl.: C07C 31/04, C07C 1/12, C10G 2/00

(54) **ENERGY INTEGRATED CARBON DIOXIDE CONVERSION PROCESS**
ENERGIEINTEGRIERTES KOHLENDIOXIDUMWANDLUNGSVERFAHREN
PROCÉDÉ DE CONVERSION DU DIOXYDE DE CARBONE À APPORT D'ÉNERGIE INTÉGRÉ

(30) Priority: 14.11.2013 US 201361903984 P
(43) Date of publication of application: 21.09.2016
(73) Proprietor: Climeworks AG, 8050 Zürich (CH)
(72) Inventor: O'CONNOR, Paul, NL-3871 KM Hoevelaken (NL); ROESTENBERG, Timo, NL-7433 HG Schalkhaar (NL); MARINIC, Sasa, NL-3892 AC Zeewolde (NL)
(74) Representative: Bremi, Tobias Hans
(86) International application number: PCT/EP2014/074688
(87) International publication number: WO 2015/071443

(56) References cited:
- WO-A1-2012/168355
- DE-A1-102006 034 712
- GB-A- 2 448 685
- US-A- 3 511 595
- US-A1- 2008 267 842
- US-B1- 6 387 337
- J.G. VAN BENNEKOM ET AL: "Methanol synthesis beyond chemical equilibrium", CHEMICAL ENGINEERING SCIENCE, vol. 87, 16 December 2012 (2012-12-16), pages 204-208, XP055161548, ISSN: 0009-2509, DOI: 10.1016/j.ces.2012.10.013

## Description

### BACKGROUND OF THE INVENTION

### 1. Field of the Invention

The invention relates generally to a process for converting carbon dioxide in an exothermic reaction, and more specifically to such process whereby energy released in the exothermic reaction is used in a preceding carbon dioxide enrichment step.

### 2. Description of the Related Art

Prior art processes for converting carbon dioxide to liquid products, such as methanol, are known. Such processes require feed gases having high carbon dioxide content. For this reason such processes generally are integrated with industrial plants that produce gases having high carbon dioxide content, such as power plants, refineries, and the like. Such gases typically contain contaminants, such as SOx, that are corrosive and act as poisons to carbon dioxide conversion catalysts. The feed gases therefore need to be purified before they can be used in the carbon dioxide conversion process.

Carbon dioxide conversion processes, such as the conversion to methanol, are strongly exothermic. Thermal energy must continuously be withdrawn from the carbon dioxide conversion reactor. To avoid energy waste and thermal pollution of the environment, carbon dioxide conversion plants need to be operated in conjunction with other industrial processes having a net energy demand, so that heat generated by the carbon dioxide conversion process can beput to good economic use.

Thus, there is a need for a carbon dioxide conversion process that is sufficiently energy balanced to be operated in stand-alone fashion. There is a further need for a carbon dioxide conversion process that can use feedstock gases that have relatively low carbon dioxide concentration, such as ambient air.

GB2448685 discloses, that carbon dioxide, derived directly or indirectly from the atmosphere (e.g. by sequestration from air using an absorbent or adsorbent material), and hydrogen, derived from water (e.g. by electrolysis), may be reacted to produce a range of organic compounds. Carbon dioxide and hydrogen can be converted into carbon monoxide and water (2) by the reverse water gas shift reaction (or reverse shift reaction). Carbon monoxide and hydrogen can be converted into methane by a methanation reaction (3), or methanol (4), or (by the Fischer-Tropsch process) a range of hydrocarbons. Using moving-bed catalysis (5), gaseous and light liquid fuels (6) such as alcohols, petrol (gasoline) and paraffin (kerosine) can be produced. Using fixed-bed catalysis (7), heavy fuels (8) such as diesel oil can be produced. Such fuels may be used for energy production, for combustion in an engine and for generating electricity in a fuel cell. Heat produced in exothermic reactions may be recovered and used to reduce the energy input requirements of the electrolytic conversion of water to hydrogen and of endothermic reactions.

Van Bennekom, et al (2013, Methanol synthesis beyond chemical equilibrium. Chemical Engineering Science, 87(11), 204-208) disclose, that in commercial methanol production from syngas, the conversion is thermodynamically limited to 0.3-0.7 leading to large recycles of non-converted syngas. This problem can be overcome to a significant extent by in situ condensation of methanol during its synthesis which is possible nowadays due to the availability of highly active catalysts allowing for lower reactor temperatures. For the first time, in situ methanol condensation at 20 MPa and 0°C was demonstrated visually in a view cell. The condensation of reaction products (mainly methanol and water) drives the equilibrium reactions nearly to completion, as is demonstrated experimentally in a packed bed reactor and supported by thermodynamic calculations. Contrary to conventional methanol synthesis, once-through operation becomes possible avoiding recycling of unconverted syngas, which can be economically beneficial for industrial stakeholders.

### BRIEF SUMMARY OF THE INVENTION

The present invention addresses these problems by providing an energy integrated C0₂ conversion process as claimed..

### DETAILED DESCRIPTION OF THE INVENTION

The following is a detailed description of the invention.

### Definitions

An important aspect of the process of the present invention is the use of a feed gas having low carbon dioxide concentration. The economic advantages of the process are most pronounced when feed gases having a low carbon dioxide concentrations are used. The process is using ambient air as a feed gas. Ambient air currently has a carbon dioxide concentration of about 400 ppm. As ambient air is abundantly available, and substantially free of corrosive contaminants, ambient air is the feed gas for the process of the present invention.

The process comprises a carbon dioxide enrichment step, whereby the carbon dioxide concentration of the feed gas is increased. The carbon dioxide enrichment step requires an energy input.

According to the invention, the carbon dioxide enrichment process is a process whereby the feed gas is contacted with an absorbent material capable of absorbing carbon dioxide. In a desorption step, absorbed carbon dioxide is released from the absorbent material in a form that is purified relative to the feed gas. Such processes require energy input for contacting the feed gas with the absorbent material, for example using a compressor to force the feed gas through a bed of absorbent material. It will be understood that, the lower the carbon dioxide concentration of the feed gas, the larger the volume of feed gas that must be contacted with the absorbent, and the larger the energy input required.

The desorption step involves heating the absorbent material, flushing the absorbent material with a flushing gas, applying vacuum to the absorbent material, or a combination of these measures. Whatever method is employed for desorbing carbon dioxide from the absorbent material, energy input will unavoidably be required.

The absorbent material is alkali metal and earth alkaline salts. It has been found that salts that absorb carbon dioxide under formation of a bicarbonate are particularly suitable. Sodium and potassium salts, in particular potassium salts, are particularly preferred, in particular sodium and potassium carbonates and hydrates thereof. Highly preferred absorbent materials are disclosed in more detail in our co-pending patent application PCT/EP 2013/065070, to which reference is made in this respect.

Swing processes have been found to be particularly suitable for enrichment of carbon dioxide-containing feed gases. The method as claimed includes a temperature swing process. A preferred method and apparatus, optionally using water vapor as a flushing gas, is disclosed in our co-pending patent application PCT/EP 2013/065074, to which disclosures reference is made in this respect.

The feed gas enriched in carbon dioxide is used in a subsequent carbon dioxide conversion reaction. The process of the invention is the conversion of carbon dioxide to methanol. A suitable example of this conversion is the thermo-catalytic conversion. Various catalysts have been proposed for this reaction, for example Cu/ZnO/Al₂O₃. See, for example, a discussion of the history of the industrial use of this reaction in J.G. van Bennekom et al., Chem. Eng. Sci. 87 (2013) 204-208.

The conversion reaction:

CO₂ + 3H₂ → CH₃OH + H₂O (1)

is exothermic, and produces 141 kJ/mole. In addition the process generally includes condensation of the reaction products (methanol and water), which produces 28 kJ mole of methanol, and 39 kJ/mole of water, for a total of 67 kJ/mole of converted C0₂.

The condensation of methanol takes place in the conversion reactor. This can be accomplished by carrying out the reaction at a relatively high pressure (e.g., 25-175 bar), and by providing one or more condensation zones inside the reactor. The condensation zone or zones are kept at a lower temperature than the reaction zone or zones inside the reactor. It will be understood that the temperature of the condensation zone or zones must be low enough to achieve condensation of methanol.

Carrying out condensation of methanol inside the reactor has the important advantage of causing an equilibrium shift. In effect, condensation of methanol causes this reaction product to be withdrawn from the reaction mixture, which shifts the equilibrium of reaction (1) to the right hand side.

In an alternate embodiment unreacted reactants and reaction products are withdrawn from the conversion reactor as a gaseous mixture. The reaction products are separated from this mixture by condensation in a separate vessel. The unreacted reactants are recycled to the conversion reactor.

The integrated process of the invention comprises various heating and cooling steps. Step a. is be carried out at temperatures in the range of -23.15°C (250K) to 176.85°C (450K). Step a. may involve a temperature swing over a range of 60°C to 150°C, preferably from 60°C to 99°C. Step b. is be carried out at a temperature in the range of 151.85°C (425K) to 276.85°C (550K). In general, increasing the temperature of reactants, reaction products and equipment requires an energy input; decreasing the temperature of reactants, reaction products and equipment produces an energy output. The process of the invention aims at capturing the energy outputs as much as possible, and applying them to the required energy inputs.

Key to successful operation of the process of the invention is the significant energy output of step b. It should be noted that step b. is the conversion of CO₂ to a liquid reaction product, that is, its energy output comprises the heat of reaction of the conversion, and the heat of condensation of the reaction products. Accordingly, step b. may produce from 1000kJ to 200kJ per mole of converted CO₂.

### DESCRIPTION OF ILLUSTRATIVE EXAMPLES

The following is a description of examples not falling under the scope of the claims.

### Example 1

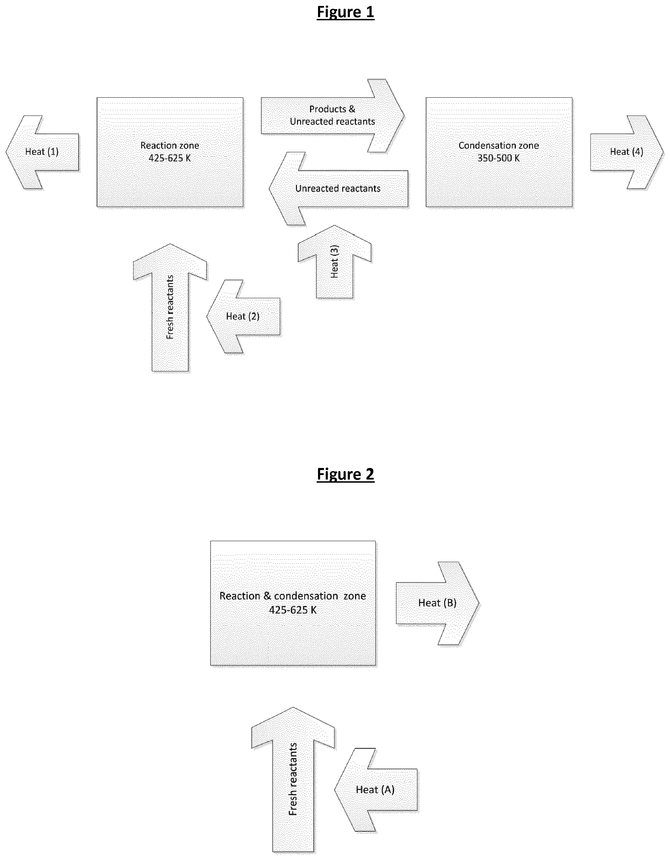

Figure 1 is a schematic representation of an embodiment of the process. The reaction zone and the condensation zone are operated at a relatively low pressure, for example in the range of 25 to 100 bar. The reaction zone and the condensation zone are kept at significantly different temperatures. The reaction zone may be at 151.85 °C (425K) to 351.85 °C (625K); the condensation zone at 76.85 °C (350K) to 226.85 °C (500K). Figure 1 also represents an example in which the condensation is carried out in a separate vessel.

It will be understood that Figure 1 captures only step b. of the process. The various energy flows are as follows. Heat (1) is the heat of reaction. Heat (2) is the energy required to raise the temperature of the reactants from ambient to the reaction temperature. In many cases carbon dioxide produced in step a. may have a temperature significantly above ambient temperature, so that Heat (2) in those cases is less than is calculated in this example.

Unreacted reactants are recycled from the condensation zone to the reaction zone. Heat (3) represents the energy input required for raising the temperature of the unreacted reactants from the condensation zone temperature to the reaction zone temperature. Heat (4) is the energy output resulting from cooling the reactants and reaction products from the reaction zone temperature to the condensation zone temperature, and the heat of condensation of the reaction products.

The energy balance has been calculated for a reaction zone temperature of 251.85 °C (525K) and a condensation zone temperature of 101.85 °C (375K). The conversion is assumed to be 20% per pass.
This means that for every mole CO₂ in the fresh reactant stream there are 4 moles of CO₂ in the stream of unreacted reactants. For every mole of CO₂ in the fresh reactant stream there are 3 moles of H₂ in the fresh reactant stream, and 12 moles of H₂ in the stream of unreacted reactants.

The heat balance calculations are summarized in Table 1

| Flow | Contributor | Energy (per mole C02 converted) | Total |
|---|---|---|---|
| Heat (1) | Reaction heat | 141 kJ @251.85°C | 141 kJ @251.85°C |
| Heat (2) | Heat capacity CO₂, 1 mole, 19.85°C (293) → 251.85°C (525K) | -9.5 kJ @251.85°C (525K) | -37 kJ@251.85°C (525K) |
| | Heat capacity H₂, 3 mole, 19.85°C (293) → 251.85°C | -27 kJ@251.85°C (525K) | |
| Heat (3) | Heat capacity CO₂, 4 mole, 101.85°C (375)→ 251.85°C (525K) | -25 kJ@251.85°C (525K) | -95 kJ@251.85°C (525K) |
| | Heat capacity H₂, 12 mole, 101.85°C (375)→ 251.85°C (525K) | -70 kJ@251.85°C (525K) | |
| Heat (4) | Heat capacity CO₂, 4 mole, 251.85°C (525)→ 101.85°C (375K) | 25 kJ@101.85°C (375K) | 176 kJ@101.85°C (375K) |
| | Heat capacity H₂, 12 mole, 251.85°C (525)→101.85°C (375K) | 70 kJ @101.85°C (375K) | |
| | Heat capacity CH₃OH, 1 mole, 251.85°C (525) | 9.2 kJ@101.85°C (375K) | |
| | Heat capacity H₂O, 1 mole, 251.85°C (525)→ 101.85°C (375K) | 5.4 kJ @101.85°C (375K) | |
| | Condensation heat CH₃OH, 1 mole | 28 kJ @101.85°C | |
| | Condensation heat H₂O, 1 mole | 39 kJ @101.85°C | |

In Table 1, positive values represent energy outputs, and negative values represent energy inputs. The net energy output of step b. in this example is 141-37-95+176=185kJ per mole of converted CO2.
Step a. may be a temperature swing absorption/desorption process, whereby CO₂ is absorbed from ambient air by potassium sesquihydrate on an active carbon support. Absorption of CO₂ involves conversion of the potassium carbonate to potassium bicarbonate, and desorption requires the reverse reaction. The heat of reaction is 41 kJ/mole. The heat capacity of the active carbon support is 900 J/kg^{∗}K. The absorption capacity of the absorbent material, which according to literature data is between 1 and 2.5 mole of CO₂ per kg, is for the purpose of this calculation assumed to be 1 mole/kg. If the available heat energy from step b. is 185 kJ/mole, the achievable temperature swing is (185-41)/0.9 = 160K. This is more than sufficient for an absorption/desorption swing process.

### Example 2

Figure 2 schematically depicts an example of the process in which the reaction zone and the condensation zone are at the same (or close to the same) temperature, for example in the range of from 425K to 625K.To accomplish methanol condensation at the reaction temperature, a relatively high pressure is required, for example >150 bar.

Heat (A) is the energy input required for raising the temperature of the reactants from ambient temperature to the reaction temperature. Heat (B) is the energy output resulting from the conversion reaction and the condensation of the reaction products.

The energy balance has been calculated for a reaction temperature of 251.85°C (525K). The one-pass conversion is assumed to be 100% (due to the equilibrium shift resulting from the *in situ* condensation of methanol). The energy calculations are summarized in Table 2.

**Table 2**

| Flow | Contributor | Energy (per mole C02 converted) | Total |
|---|---|---|---|
| Heat (A) | Heat capacity CO₂, 1 mole, 19.85°C (293)→ 251.85°C (525K) | -9.5 kJ @251.85°C (525K) | -37 kJ@251.85°C (525K) |
| | Heat capacity H₂, 3 mole, 19.85°C (293)→ 251.85°C | -27 kJ@251.85°C (525K) | |
| Heat (B) | Reaction heat | 141 kJ @251.85°C | 208 kJ @251.85°C |
| | Condensation heat CH₃O H, 1 mole | 28 kJ @101.85°C | |
| | Condensation heat H₂O, 1 mole | 39 kJ@101.85°C | |

In this example step b. produces (208-37)=171kJ/mole of converted CO₂. As in Example 1, the energy produced by step b. is more than the energy required for step a.

It will be recognized that these examples are susceptible to various modifications and alternative forms well known to those of skill in the art.

Many modifications in addition to those described above may be made to the structures and techniques described herein. Accordingly, these are examples only and are not limiting upon the scope of the invention.

## Claims

1. An energy integrated CO2 conversion process comprising the steps of
a. a CO2 enrichment step whereby the CO2 concentration of a feed gas in the form of ambient air is increased by using an absorbent comprising an alkali metal salt or an alkaline earth salt, said enrichment step requiring an energy input;
b. a CO2 conversion step whereby CO2 is converted to a liquid product by hydrogenation of carbon dioxide to methanol, said conversion step producing an energy output;
whereby the energy output is used to partially offset the energy input,
wherein step a. comprises a swing absorption/desorption process comprising a temperature swing,
wherein step a. is operated at temperatures in a range of from -23.15°C (250K) to 176.85°C (450K) and
wherein step b. is operated at temperatures in a range of from 151.85°C (425K) to 276.85°C (550K)
wherein step b. is carried out in a reactor, and equilibrium shift is accomplished by condensing methanol inside the reactor,
wherein the reactor comprises at least one reaction zone and at least one methanol condensation zone, the reaction zone or zones having a higher temperature than the methanol condensation zone or zones.

2. The process of claim 1 wherein the absorbent comprises a salt of sodium or potassium, preferably potassium, preferably potassium carbonate or a hydrate thereof.

3. The process of any one of the preceding claims wherein step b. comprises a thermo-catalytic conversion of CO2 to methanol.

4. The process of any one of the preceding claims wherein the reaction zone or zones have substantially the same pressure as the condensation zone or zones.

5. The process of any one of the preceding claims wherein step a. comprises a temperature swing over a range of 60°C to 150°C.

6. The process of claim 5 wherein step a. comprises a temperature swing over a range of 60°C to 99°C.

7. The process of any one of the preceding claims wherein step b. produces from 100 kJ to 200 kJ per mole of converted CO2.

## Patentansprüche

1. Ein energieintegrierter CO2-Umwandlungsprozess, der die folgenden Schritte umfasst
a. einen CO2-Anreicherungsschritt, bei dem die CO2-Konzentration eines Zufuhrgases in Form von Umgebungsluft unter Verwendung eines Absorptionsmittels, das ein Alkalimetallsalz oder ein Erdalkalimetallsalz umfasst, erhöht wird, wobei der Anreicherungsschritt eine Energiezufuhr erfordert;
b. einen CO2-Umwandlungsschritt, bei dem CO2 durch Hydrierung von Kohlendioxid zu Methanol in ein flüssiges Produkt umgewandelt wird, wobei der Umwandlungsschritt eine Energieabgabe erzeugt;
wobei die Energieabgabe verwendet wird, um die Energiezufuhr teilweise auszugleichen,
wobei Schritt a. einen Swing-Absorptions-/Desorptionsprozess umfasst, der einen Temperaturwechsel umfasst,
wobei Schritt a. bei Temperaturen in einem Bereich von -23,15°C (250K) bis 176,85°C (450K) betrieben wird und
wobei Schritt b. bei Temperaturen in einem Bereich von 151,85°C (425K) bis 276,85°C (550K) betrieben wird
wobei Schritt b. in einem Reaktor durchgeführt wird und die Gleichgewichtsverschiebung durch Kondensation von Methanol im Inneren des Reaktors erreicht wird,
wobei der Reaktor mindestens eine Reaktionszone und mindestens eine Methanolkondensationszone umfasst, wobei die Reaktionszone(n) eine höhere Temperatur als die Methanolkondensationszone(n) aufweist (aufweisen).

2. Verfahren nach Anspruch 1, wobei das Absorptionsmittel ein Salz von Natrium oder Kalium, vorzugsweise Kalium, vorzugsweise Kaliumcarbonat oder ein Hydrat davon umfasst.

3. Verfahren nach einem der vorstehenden Ansprüche, wobei Schritt b. eine thermo-katalytische Umwandlung von CO2 in Methanol umfasst.

4. Verfahren nach einem der vorstehenden Ansprüche, wobei die Reaktionszone oder -zonen im Wesentlichen den gleichen Druck wie die Kondensationszone oder -zonen haben.

5. Verfahren nach einem der vorstehenden Ansprüche, wobei Schritt a. einen Temperaturwechsel über einen Bereich von 60°C bis 150°C umfasst.

6. Verfahren nach Anspruch 5, wobei Schritt a. einen Temperaturwechsel über einen Bereich von 60°C bis 99°C umfasst.

7. Verfahren nach einem der vorhergehenden Ansprüche, wobei Schritt b. 100 kJ bis 200 kJ pro Mol umgewandeltes CO2 erzeugt.

## Revendications

1. Un procédé de conversion de CO2 intégré à l'énergie comprenant les étapes suivantes
a. une étape d'enrichissement en CO2 par laquelle la concentration en CO2 d'un gaz d'alimentation sous la forme d'air ambiant est augmentée en utilisant un absorbant comprenant un sel de métal alcalin ou un sel alcalino-terreux, ladite étape d'enrichissement nécessitant un apport d'énergie ;
b. une étape de conversion du CO2 par laquelle le CO2 est converti en un produit liquide par hydrogénation du dioxyde de carbone en méthanol, ladite étape de conversion produisant une production d'énergie ;
la production d'énergie étant utilisée pour compenser partiellement les apports d'énergie,
dans lequel l'étape a. comprend un procédé d'absorption/désorption à swing comprenant un swing de température,
dans lequel l'étape a. est mise en oeuvre à des températures dans une plage de -23,15°C (250K) à 176,85°C (450K) et
dans lequel l'étape b. est mise en oeuvre à des températures dans une plage de 151,85°C (425K) à 276,85°C (550K)
dans lequel l'étape b. est effectuée dans un réacteur, et le changement d'équilibre est accompli par condensation du méthanol à l'intérieur du réacteur,
dans lequel le réacteur comprend au moins une zone de réaction et au moins une zone de condensation du méthanol, la ou les zones de réaction ayant une température plus élevée que la ou les zones de condensation du méthanol.

2. Procédé selon la revendication 1, dans lequel l'absorbant comprend un sel de sodium ou de potassium, de préférence de potassium, de préférence du carbonate de potassium ou un hydrate de celui-ci.

3. Procédé selon l'une quelconque des revendications précédentes, dans lequel l'étape b. comprend une conversion thermo-catalytique de CO2 en méthanol.

4. Procédé selon l'une quelconque des revendications précédentes, dans lequel la ou les zones de réaction ont sensiblement la même pression que la ou les zones de condensation.

5. Procédé selon l'une quelconque des revendications précédentes, dans lequel l'étape a. comprend une variation de température sur une plage de 60°C à 150°C.

6. Procédé selon la revendication 5, dans lequel l'étape a. comprend une variation de température sur une plage de 60°C à 99°C.

7. Procédé selon l'une quelconque des revendications précédentes, dans lequel l'étape b. produit de 100 kJ à 200 kJ par mole de CO2 converti.
